# EUROPEAN PATENT SPECIFICATION

(11) **EP 1 044 959 B1**
(45) Date of publication and mention of the grant of the patent: **04.12.2002**
(21) Application number: 00303078.0
(22) Date of filing: 12.04.2000
(51) Int. Cl.: C07C 209/52, C07C 211/15

(54) **Process for preparing a 1,1,1-trifluoro-2-aminoalkane**
Verfahren zur Herstellung von 1,1,1-Trifluoro-2-aminoalkane
Procédé pour la préparation de 1,1,1-trifluoro-2-aminoalkanes

(30) Priority: 15.04.1999 US 292687
(43) Date of publication of application: 18.10.2000
(73) Proprietor: BASF AKTIENGESELLSCHAFT, 67056 Ludwigshafen (DE)
(72) Inventor: Eisenacht, Rudi, 55131 Mainz (DE); Niedermann, Hans-Peter, 55270 Bubenheim (DE); Landau, Dieter, 55237 Flonheim (DE)
(74) Representative: Langfinger, Klaus Dieter, Dr.

(56) References cited:
- ONO, TAIZO ET AL: "Biomimetic Reductive Amination of Fluoro Aldehydes and Ketones via [1,3]-Proton Shift Reaction. Scope and Limitations" J. ORG. CHEM. (1996), 61(19), 6563-6569 , XP002146990

## Description

### BACKGROUND OF THE INVENTION

The invention relates to a novel process for the preparation of 1,1,1-trifluoro-2-aminoalkanes which comprises reacting the corresponding N-alkylidene benzylamines with a primary amine whereby the product compound is removed by distillation.

1,1,1-trifluoro-2-aminoalkanes are useful as intermediates for the preparation of a variety of compounds which are useful as agrochemicals, pharmaceuticals or dyes. In particular, they are key intermediates in the preparation of insecticidal benzamides as disclosed for example by DE 36 11 193 and of fungicidal 7-(1,1,1-trifluoroalk-2-ylamino)-6-(halophenyl)-triazolopyrimidines which are described for example in WO 98/46608.

T. Ono, et al., J. Org. Chem. **61,** 1996, 6563-6569 disclose a method for the preparation of 1,1,1-trifluoro-2-aminopropane by rearrangement of N- (1,1,1-triflluoroisopropylidene)benzylamine to N-benzylidene-1,1,1-trifiluoroisopropylamine. The resulting benzylidene compound is hydrolyzed to the amine hydrochloride with hydrochloric acid using ether as diluent. The amine hydrochloride is separated from the benzaldehyde and the free amine is prepared by action of triethylamine on the amine hydrochloride.

Thus the process known from the art requires several steps to yield the desired 1,1,1-trifluoro-2-aminoalkanes. The novel process has been found to be advantageous in producing 1,1,1-trifluoro-2-aminoalkanes in only up to two steps.

### SUMMARY OF THE INVENTION

The 1,1,1-trifluoro-2-aminoalkanes of formula I 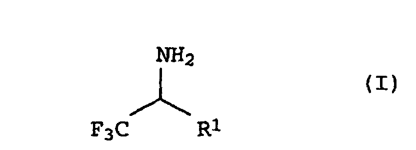 wherein
R¹ represents an optionally substituted alkyl group;
can be obtained in high yields by heating a mixture comprising or consisting essentially of a compound of formula II 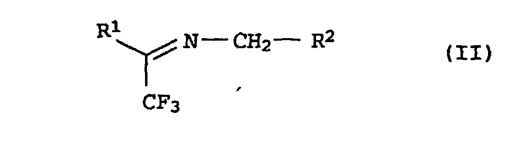 wherein
R¹ has the meaning given, and
R² represents an optionally substituted aryl group;
a primary amine, and a base which is a tertiary amine, and/or an inert diluent, whereby the compound of formula I is removed by distillation during the heating procedure.

It is an object of the present invention to provide an efficient new process for the preparation of 1,1,1-trifluoro-2-aminoalkanes of formula I.

Other objects and advantages of the present invention will be apparent to those skilled in the art from the following description and the appended claims.

### DETAILED DESCRIPTION OF THE PREFERRED EMBODIMENTS

The invention relates to a novel process for the preparation of the compounds of formula I by heating a mixture consisting essentially of a compound of formula II, a primary amine and optionally a base and/or an inert diluent. The advantage of the novel process is, that the compound of formula I is obtained directly in high yields and high purity by distillation during the heating procedure.

In general terms, unless otherwise stated herein, the term alkyl or alkoxy as used herein with respect to a radical or moiety refers to a straight or branched chain radical or moiety. As a rule, such radicals have up to 10, in particular up to 6 carbon atoms. Suitably an alkyl or alkoxy moiety has from 1 to 6 carbon atoms, preferably from 1 to 3 carbon atoms. A particularly preferred alkyl moiety is the methyl group.

In general terms, unless otherwise stated herein, the term aryl as used herein with respect to a radical or moiety refers to an aryl group having 6, 10 or 14 carbon atoms, preferably 6 or 10 carbon atoms. A particularly preferred aryl moiety is the phenyl group being optionally substituted by one or more halogen atoms, nitro, cyano, alkyl, preferably C₁₋₆ alkyl, alkoxy, preferably C₁₋₆ alkoxy.

Suitable primary amines are primary amines having 2 to 20 carbon atoms, preferably 6 to 14 carbon atoms. Preferred are primary amines having a boiling point higher than the boiling point of the compound of formula I, in particular aralkylamines being optionally substituted by one or more halogen atoms, alkyl, preferably C₁₋₆ alkyl, alkoxy, preferably C₁₋₆ alkoxy, most preferably benzylamine.

Preferably the reaction is carried out with a compound of formula II to primary amine molar ratio from 1 : 1 to 1 : 1.5, in particular from 1 : 1.1 to 1 : 1.3, most preferred at a ratio of about 1 : 1.2.

Suitable inert diluents are cyclic ethers, aliphatic ethers, aliphatic hydrocarbons or aromatic hydrocarbons. Most preferred are aromatic hydrocarbons having 6 to 14 carbon atoms, in particular benzene, toluene, xylene or mesitylene.

Suitable bases are tertiary amines, in particular bicyclic tertiary amines, such as 1,8-diazabicyclo [5.4.0]undecene-7 (DBU) or 1,5-diazabicyclo [3.4.0]nonene-5 (DBN).

Preferably the reaction is carried out in the presence of catalytic amounts of a base, in particular at a compound of formula II to base molar ratio from 1 : 0.001 to 1 0.1, most preferred from 1 : 0.005 to 1: 0.05.

A preferred embodiment of the present invention is a process wherein one or more (eg all) the following apply:
- the reaction is carried out at temperatures between 80 and 140 °.C; preferably between 90 °C and 130 °C, in particular between 95 °C and 120 °C; most preferred at about 115 °C; and/or
- the reaction is carried out at atmospheric pressure; and/or
- the reaction mixture consists of a compound of formula II, a catalytic amount of a base, preferably DBU, and/or
- R¹ represents a C₁₋₄ alkyl group being optionally substituted by one or more halogen atoms or an alkoxycarbonyl or hydroxycarbonyl group, in particular wherein R¹ represents a methyl group.

The compound of formula II is most preferably N-(1,1,1-trifluoro-isopropylidene)benzylamine. The compound of formula II can be prepared by a condensation reaction between commercially available 1,1,1-trifluoro-2-oxoalkane and benzylamine or a salt thereof as disclosed for example T. Ono, et al., loc.cit.

In a preferred embodiment of the invention the preparation of the compound of formula I is carried out in a one-pot synthesis comprising the steps of
a) condensation of a 1,1,1-trifluoro-2-oxo-alkane of formula III, 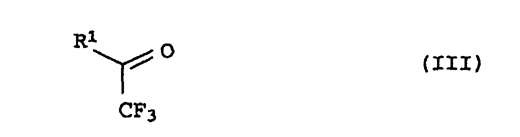 and an optionally substituted benzylamine, of formula IV,

   H₂N ― CH₂ ― R² (IV)

   preferably by azeotropic removal of the water formed during the reaction with the aid of an inert aromatic diluent in the presence of an acid, preferably p-toluenesulfonic acid, and
b) treating the resulting N-(1,1,1-trifluoroalkyliden-2-yl)benzylamine with a primary amine in the presence of a base which is a tertiary amine, and/or an inert aromatic diluent at elevated temperatures, whereby the desired compound of formula I distills off.

After the condensation step (a), preferably using mixture of mesitylene and toluene as an inert diluent in the presence of p-toluenesulfonic acid the reaction water together optionally with the low boiling diluent, preferably toluene, is separated via a water separator. An excess the primary amine and catalytic amounts of the base, preferably benzylamine and DBU are added at a temperature from 70°C to 120°C, in particular at about 90°C and 5 the liberated compound of formula I is rectificated over a column and collected in an ice cooled trap.

The crude product obtained can be purified according to standard methods for example by distillation, recrystallization or chromatographic methods.

However, the crude product obtained according to the process of this invention is as a rule pure enough to be used as intermediate without further purification.

In a particularly preferred embodiment of the process according to this invention a mixture of the compound of formula II, in particular N-(1,1,1-trifluoroisopropylidene)benzylamine, a primary amine, preferably benzylamine and a catalytic amount of a base, preferably DBU is heated at a temperature of about 115 °C. The amine of formula I, in particular 2-amino-1,1,1-trifluoropropane, is obtained by distillation during the reaction. The reaction temperature of about 115 °C is kept until no further amine distills off.

In order to facilitate a further understanding of the invention, the following illustrative examples are presented.

### Example 1

### Preparation of N-(1,1,1-trifluoroisopropylidene)benzylamine

3360 g of 1,1,1-trifluoroacetone were added to a solution of 1,5 g of p-toluenesulfonic acid in 10 I toluene after cooling down to 0 °C. Subsequently, 3225 g of benzylamine were dosed within 3 hours at a temperature range of 0 to 10°C. The reaction mixture was heated with reflux for 15 hours. The reaction water was distilled off and subsequently the mixture cooled to room temperature. The solvent was removed in vacuo to yield 5420 g of a crude mixture containing 71,7 % of N-(1,1,1-trifluoroisopropylidene) benzylamine. The mixture was used for the preparation of 2-amino-1,1,1-trifluoropropane without further purification.

### Example 2

### Preparation of 2-amino-1,1,1-trifluoropropane

4643 g of N-(1,1,1-trifluoroisopropylidene) benzylamine obtained in Example 1 were placed in a three necked flask with stirrer, dropping funnel and descending condenser with an ice-cooled flask. 2755 g of benzylamine were added and the mixture was heated up to 90°C. 20 ml of DBU were added and the 2-amino-1,1,1-trifluoropropane started to distill off. The reaction mixture was heated up to 115 °C and subsequently 25 ml of DBU were added. The reaction temperature was kept at 115 °C until no further 2-amino-1,1,1-trifluoropropane distilled off. 1857 g of the pure product were collected having a boiling point of 46-47 °C.

### Example 3

### Preparation of 2-amino-1,1,1-trifluoropropane

In a three necked flask with reflux condenser and dropping funnel a mixture of 300 ml toluene, 200 ml mesitylene, 112 g 1,1,1-trifluoroacetone and 0,5g of p-toluene sulfonic acid was cooled to 0°C with magnetical stirring. To this mixture 112,5 g benzylamine was added dropwise keeping the temperature below 10°C. The reaction mixture was heated to reflux over night. After cooling to room temperature the reflux condenser was substituted by a Dean-Stark trap and the mixture heated to reflux to separate the reaction water.

After separation of the reaction water the mixture was cooled to room temperature and the Dean-Stark trap was substituted by a rectification unit. A mixture of 153 g benzylamine and 2 ml DBU was slowly added and the mixture was heated to 95°C. 2-Amino-1,1,1-trifluoropropane distilled of and was collected in an ice cooled trap. Another 28 ml of DBU was added portionwise until no 2-amino-1,1,1-trifluoropropane distilled off.
Yield: 77 g.

## Claims

1. A process for the preparation of a 1,1,1-trifluoro-2-aminoalkane of formula I 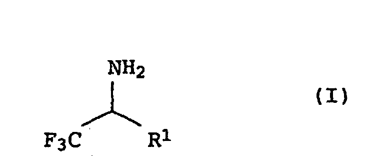 wherein
R¹ represents an optionally substituted alkyl group;
which comprises
heating a mixture of a compound of formula II 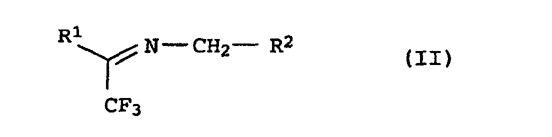 wherein
R¹ has the meaning given, and
R² represents an optionally substituted aryl group;
a primary amine, and a base which is a tertiary amine, and/or an inert diluent, whereby the compound of formula I is removed by distillation during the heating procedure.

2. A process according to claim 1, wherein the reaction is carried out at a temperature from 80°C to 140°C.

3. A process according to claim 1 or claim 2, wherein the reaction is carried out at atmospheric pressure.

4. A process according to any one of claims 1 to 3, wherein the diluent is an optionally substituted aromatic hydrocarbon, preferably toluene, xylene or mesitylene.

5. A process according to any one of claims 1 to 4, wherein the compound of formula II to primary amine molar ratio is from 1:1 to 1:1,5.

6. A process according to any one of claims 1 to 5, wherein the primary amine has a boiling point higher than the boiling point of the compound of formula I.

7. A process according to any one of claims 1 to 6, wherein the primary amine is an optionally substituted benzylamine.

8. A process according to claim 1, wherein the tertiary amine is 1,8-diazabicyclo[5.4.0]undecene-7 (DBU).

9. A process according to claim 1, which comprises the steps of:
a) condensation of a 1,1,1-trifluoro-2-oxo-alkane of formula III, 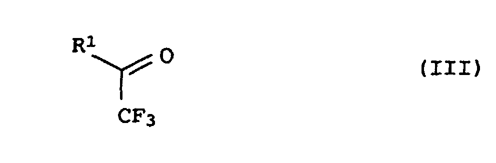 wherein R¹ has the meaning given, and an optionally substituted benzylamine of formula IV,
H₂N ― CH₂ ― R² (IV)
wherein R² has the meaning given, by azeotropic removal of the water formed during the reaction with the aid of an inert aromatic diluent; and
b) treating the resulting N-(1,1,1-trifluoroalkyliden-2-yl)benzylamine with a primary amine in the presence of a base and/or an inert aromatic diluent at elevated temperatures, whereby the desired compound of formula I distills off.

10. A process according to any one of claims 1 to 9, wherein R¹ represents a C₁₋₄ alkyl group being optionally substituted by one or more halogen atoms or an alkoxycarbonyl group.

11. A process according to claim 10, wherein R¹ represents a methyl group.

## Patentansprüche

1. Verfahren zur Herstellung eines 1,1,1-Trifluor-2-aminoalkans der Formel I 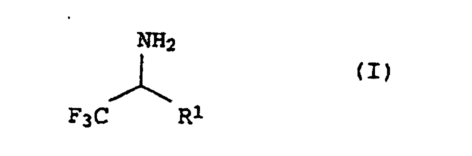 worin
R¹ für eine gegebenenfalls substituierte Alkylgruppe steht;
**dadurch gekennzeichnet, daß** man eine Mischung aus einer Verbindung der Formel II 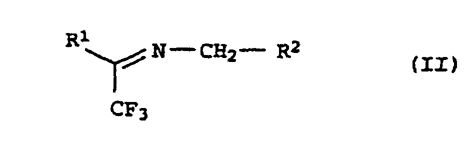 worin
R¹ die angegebene Bedeutung besitzt und
R² für eine gegebenenfalls substituierte Arylgruppe steht;
einem primären Amin und einer Base, bei der es sich um ein tertiäres Amin handelt, und/oder einem inerten Verdünnungsmittel erhitzt, wodurch die Verbindung der Formel I während des Erhitzungsvorgangs abdestilliert wird.

2. Verfahren nach Anspruch 1, bei dem man die Umsetzung bei einer Temperatur von 80°C bis 140°C durchführt.

3. Verfahren nach Anspruch 1 oder 2, bei dem man die Umsetzung bei Normaldruck durchführt.

4. Verfahren nach einem der Ansprüche 1 bis 3, bei dem man als Verdünnungsmittel einen gegebenenfalls substituierten aromatischen Kohlenwasserstoff, vorzugsweise Toluol, Xylol oder Mesitylen, einsetzt.

5. Verfahren nach einem der Ansprüche 1 bis 4, bei dem das Molverhältnis von Verbindung der Formel II zu primärem Amin 1:1 bis 1:1,5 beträgt.

6. Verfahren nach einem der Ansprüche 1 bis 5, bei dem das primäre Amin einen höheren Siedepunkt als die Verbindung der Formel I aufweist.

7. Verfahren nach einem der Ansprüche 1 bis 6, bei dem man als tertiäres Amin ein gegebenenfalls substituiertes Benzylamin einsetzt.

8. Verfahren nach Anspruch 1, bei dem man als tertiäres Amin 1,8-Diazabicyclo[5.4.0]undecen-7 (DBU) einsetzt.

9. Verfahren nach Anspruch 1, bei dem man:
a) ein 1,1,1-Trifluor-2-oxoalkan der Formel III 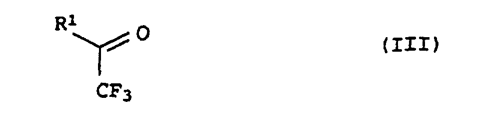 worin R¹ die angegebene Bedeutung besitzt, und ein gegebenenfalls substituiertes Benzylamin der Formel IV
**H**_{**2**}**N ― CH**_{**2**} **― R**^{**2**} **(IV)**
worin R² die angegebene Bedeutung besitzt, durch azeotrope Entfernung des bei der Reaktion gebildeten Wassers mit Hilfe eines inerten aromatischen Verdünnungsmittels kondensiert und
b) das erhaltene N-(1,1,1-Trifluoralkyliden-2-yl)-benzylamin in Gegenwart einer Base und/oder eines inerten aromatischen Verdünnungsmittels bei erhöhten Temperaturen mit einem primären Amin behandelt, wodurch die gewünschte Verbindung der Formel I abdestilliert.

10. Verfahren nach einem der Ansprüche 1 bis 9, bei dem R¹ für eine gegebenenfalls durch ein oder mehrere Halogenatome oder eine Alkoxycarbonylgruppe substituierte C₁₋₄-Alkylgruppe steht.

11. Verfahren nach Anspruch 10, bei dem R¹ für eine Methylgruppe steht.

## Revendications

1. Procédé pour la préparation d'un 1,1,1-trifluoro-2-aminoalcane de formule I 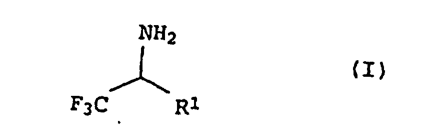 dans laquelle
R¹ représente un groupe alkyle facultativement substitué ; qui comprend le chauffage d'un mélange d'un composé de formule II 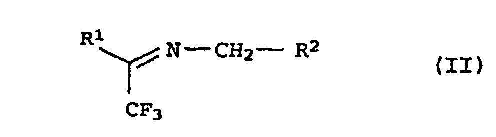 dans laquelle
R¹ est tel que défini ci-dessus, et
R² représente un groupe aryle facultativement substitué ;
d'une amine primaire et d'une base qui est une amine tertiaire, et/ou d'un diluant inerte, en sorte que le composé de formule I soit retiré par distillation pendant l'opération de chauffage.

2. Procédé selon la revendication 1, dans lequel la réaction est conduite à une température comprise entre 80°C et 140°C.

3. Procédé selon la revendication 1 ou la revendication 2, dans lequel la réaction est conduite à la pression atmosphérique.

4. Procédé selon l'une quelconque des revendications 1 à 3, dans lequel le diluant est un hydrocarbure aromatique facultativement substitué, de préférence le toluène, le xylène ou le mésitylène.

5. Procédé selon l'une quelconque des revendications 1 à 4, dans lequel le rapport molaire du composé de formule II à l'amine primaire est de 1:1 à 1:1,5.

6. Procédé selon l'une quelconque des revendications 1 à 5, dans lequel l'amine primaire a un point d'ébullition supérieur au point d'ébullition du composé de formule I.

7. Procédé selon l'une quelconque des revendications 1 à 6, dans lequel l'amine primaire est une benzylamine facultativement substituée.

8. Procédé selon la revendication 1, dans lequel l'amine tertiaire est le 1,8-diazabicyclo[5.4.0]undécène-7 (DBU).

9. Procédé selon la revendication 1, qui comprend les étapes suivantes :
a) condensation d'un 1,1,1-trifluoro-2-oxo-alcane de formule III 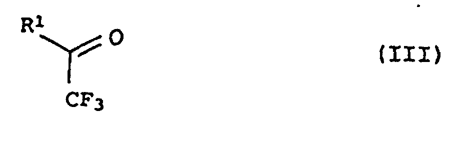 dans laquelle R¹ est tel que défini ci-dessus, et d'une benzylamine facultativement substituée de formule IV,
H₂N-CH₂-R² (IV)
dans laquelle R² est tel que défini ci-dessus, par élimination azéotropique de l'eau formée pendant la réaction à l'aide d'un diluant aromatique inerte ; et
b) traitement de la N-(1,1,1-trifluoroalkylidène-2-yl)-benzylamine résultante avec une amine primaire en présence d'une base et/ou d'un diluant aromatique inerte à des températures élevées, en sorte que le composé désiré de formule I soit retiré par distillation.

10. Procédé selon l'une quelconque des revendications 1 à 9, dans lequel R¹ représente un groupe alkyle en C₁-C₄ facultativement substitué par un ou plusieurs atomes d'halogène ou un groupe alcoxycarbonyle.

11. Procédé selon la revendication 10, dans lequel R¹ représente un groupe méthyle.
